# EUROPÄISCHE PATENTSCHRIFT

(11) **EP 3 354 190 B1**
(45) Veröffentlichungstag und Bekanntmachung des Hinweises auf die Patenterteilung: **05.12.2018**
(21) Anmeldenummer: 17153558.6
(22) Anmeldetag: 27.01.2017
(51) Int. Cl.: A61B 3/10, G02C 13/00

(54) **COMPUTERIMPLEMENTIERTES VERFAHREN ZUR DETEKTION EINES HORNHAUTSCHEITELS**
COMPUTER-IMPLEMENTED METHOD FOR DETECTING A CORNEA VERTEX
PROCÉDÉ MIS EN UVRE PAR ORDINATEUR DESTINÉ À LA DÉTECTION D'UN SOMMET DE LA CORNÉE

(43) Veröffentlichungstag der Anmeldung: 01.08.2018
(73) Patentinhaber: Carl Zeiss Vision International GmbH, 73430 Aalen (DE); Carl Zeiss AG, 73447 Oberkochen (DE)
(72) Erfinder: NIEUWENHUIS, Claudia, 73457 Essingen (DE); SCHWARZ, Oliver, 73479 Ellwangen (DE); GAMPERLING, Michael, 89340 Leipheim (DE)
(74) Vertreter: Patentanwälte Bregenzer und Reule Partnerschaftsgesellschaft mbB

(56) Entgegenhaltungen:
- JP-A- 2007 206 211
- US-A1- 2003 081 173
- US-A1- 2003 123 026
- US-A1- 2007 195 266
- US-A1- 2011 007 269

## Beschreibung

Die Erfindung betrifft ein computerimplementiertes Verfahren zur Detektion eines Hornhautscheitels gemäß Oberbegriff des Anspruchs 1.

Die Hornhautscheitel eines Probanden sind wichtige Punkte bei der Bestimmung von Zentrierparametern für die Brillenanpassung. Zentrierparameter werden benutzt, um Brillengläser korrekt in einer Brillenfassung anzuordnen bzw. zu zentrieren, so dass die Brillengläser in korrekter Position relativ zu den Augen der die Brille tragenden Person angeordnet sind. Dabei handelt es sich zum Teil um anatomische Parameter der betreffenden Person, wie beispielsweise den Pupillenabstand, zum Teil um rein fassungsspezifische Parameter wie die Fassungsscheibenbreite oder die Fassungsscheibenhöhe und zum Teil um Kombinationen aus anatomischen und fassungsspezifischen Parametern, wie beispielsweise den Hornhautscheitelabstand und die Durchblickshöhe. Einen Überblick über die gängigen Zentrierparameter gibt die DIN EN ISO 13666 vom Oktober 2013.

Eine zumindest näherungsweise Bestimmung der Position des Hornhautscheitels im Raum und vorzugsweise der Position beider Hornhautscheitel im Raum kann bei einer weiteren Auswertung vorteilhaft für die Bestimmung von Zentrierparametern, insbesondere für die Bestimmung des Hornhautscheitelabstands herangezogen werden. Bisher wurden die Hornhautscheitelpositionen manuell in den Front- und Seitenbildern annotiert. Dabei wurde je nach Vorliebe des Optikers eine reflexbasierte oder pupillenbasierte Annotation favorisiert. Bei der reflexbasierten Annotation wird mit Hilfe einer LED ein Licht-Reflex auf der Pupille erzeugt, der leichter zu detektieren ist als die Pupillenmitte. Viele Optiker bevorzugen aber nach wie vor die pupillenbasierte Annotation.
Ein Verfahren der eingangs genannten Art ist aus der US 2011/0007269 A1 bekannt.
Es ist Aufgabe der Erfindung, ein Verfahren bereitzustellen, mit dem die Position eines Hornhautscheitels im Raum genauer detektiert werden kann.

Mit dem erfindungsgemäßen Verfahren gemäß Anspruch 1 wird eine Voraussetzung dafür geschaffen, Zentrierparameter wie die Pupillendistanz und den Hornhautscheitelabstand zu berechnen, und zwar sowohl bei 'realer' Zentrierung mit einer realen Brillenfassung, als auch bei virtueller Zentrierung, wobei dem Bild eines Probanden virtuell eine Brille oder Brillenfassung "aufgesetzt" wird, wenn deren geometrische Daten vorliegen. Dabei werden vorzugsweise gleichzeitig aufgenommene kalibrierte Bilder bereitgestellt. Deren Kalibrierung umfasst die extrinsischen Eigenschaften der die Bilder aufnehmenden Kameras oder der die Bilder nacheinander aufnehmenden Kamera wie die relative Ausrichtung ihrer optischen Achsen sowie die relative Anordnung zueinander im Raum, sowie ihre intrinsischen Eigenschaften, also die Eigenschaften der Kameras selbst, die definieren, wie ein Punkt im Raum, der sich im internen Koordinatensystem der jeweiligen Kamera befindet, auf die Koordinaten der Pixel des aufgenommenen Bildes abgebildet wird. Eine ausführliche Beschreibung der Kalibrierung von Kameras findet sich im Lehrbuch "Multiple View Geometry in Computer Vision" von Richard Hartley und Andrew Zisserman, 2. Auflage, Cambridge University Press 2004, und dort insbesondere auf Seite 8. Dabei wird bevorzugt, dass bei der geometrischen Positionsbestimmung des Hornhautscheitels im Raum ein Triangulationsverfahren verwendet wird.
Die in einer ersten Näherung bestimmte Position des Hornhautscheitels im Raum wird einer Korrekturrechnung unterzogen. Die Art der Korrekturrechnung ist dann abhängig von der Art, wie die Position des Hornhautscheitels im Raum in der ersten Näherung bestimmt wird.
Es ist vorgesehen, bei einer sogenannten pupillenbasierten Auswertung die Position des Hornhautscheitels im Raum in erster Näherung als Schnittpunkt eines die Hornhaut tangierenden Sichtstrahls einer das Seitenbild aufnehmenden Seitenkamera mit einem auf die Pupille gerichteten Sichtstrahl einer das Frontbild aufnehmenden Frontkamera zu bestimmen. Erfindungsgemäß wird mittels der Korrekturrechnung die Position des Hornhautscheitels gemäß a = q + µ * v + µ * w berechnet, wobei a der Positionsvektor des Hornhautscheitels im Raum nach Durchführung der Korrekturrechnung, q die Position des Hornhautscheitels in erster Näherung, µ ein Erfahrungswert oder ein tatsächlicher Wert für den Abstand zwischen dem Pupillenzentrum und dem Hornhautscheitel, v ein Einheitsvektor der Raumrichtung vom Pupillenzentrum zur Frontkamera und w ein Einheitsvektor der durch das Zentrum der Hornhautkugel verlaufenden Blickrichtung ist.

Vorteilhaft wird die Pupille mittels Merkmalsextraktion und/oder Merkmalsmatchings (Merkmalsvergleich) und/oder mittels maschinellen Lernens durch Vergleich mit einer Vielzahl vorbekannter Daten detektiert. Diesem Verfahrensschritt kann eine Gesichtsdetektion und/oder eine Detektion von Gesichtsmerkmalen wie den Augen als Vorverarbeitung vorausgehen, bei der detektiert wird, welche Bilddaten zum Gesicht des Probanden gehören, so dass nur diese Daten in die Detektion einfließen. Zur weiteren Vereinfachung des Verfahrens kann vorgesehen sein, dass aus dem Frontbild ein auf den Hornhautscheitel gerichteter Sichtstrahl der Frontkamera 16a berechnet und in das Seitenbild projiziert wird und dass der Hornhautscheitel auf dem ins Seitenbild projizierten Sichtstrahl detektiert wird.
Um die Bestimmung des Hornhautscheitels im Raum für beide Augen vornehmen zu können, wird bevorzugt, dass mindestens ein gleichzeitig mit dem ersten und zweiten Bild seitlich bezüglich des Kopfes aufgenommenes, kalibriertes drittes Bild bereitgestellt wird.

Vorzugsweise wird das erfindungsgemäße computerimplementierte Verfahren mit einer Vorrichtung durchgeführt, wie sie grundsätzlich in Anspruch 8 und im Detail in der folgenden Figurenbeschreibung beschrieben wird.

Im Folgenden wird die Erfindung anhand eines in der Zeichnung schematisch dargestellten Ausführungsbeispiels näher erläutert. Es zeigen:

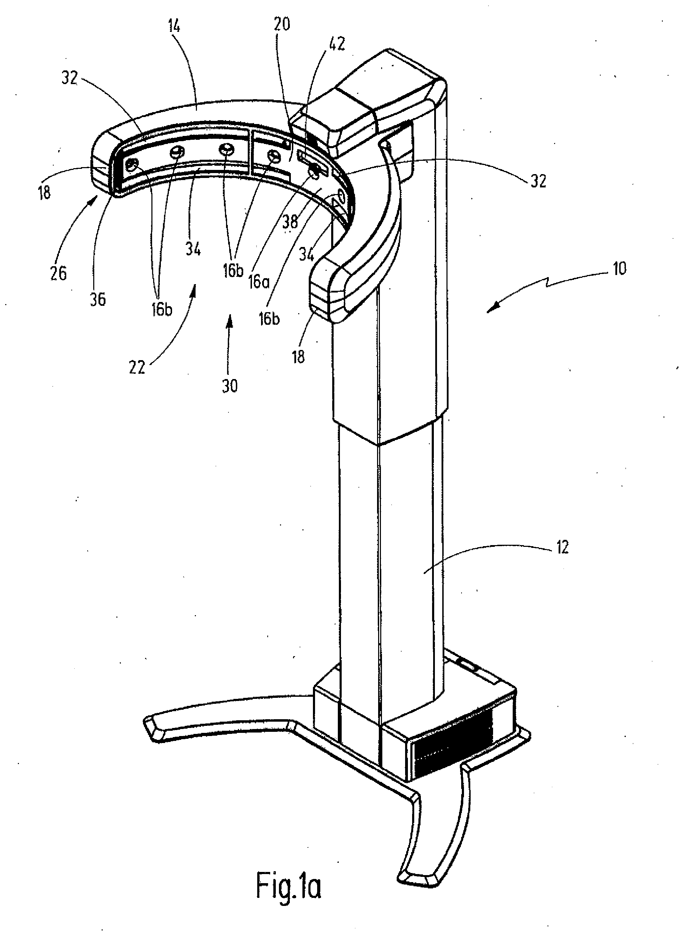

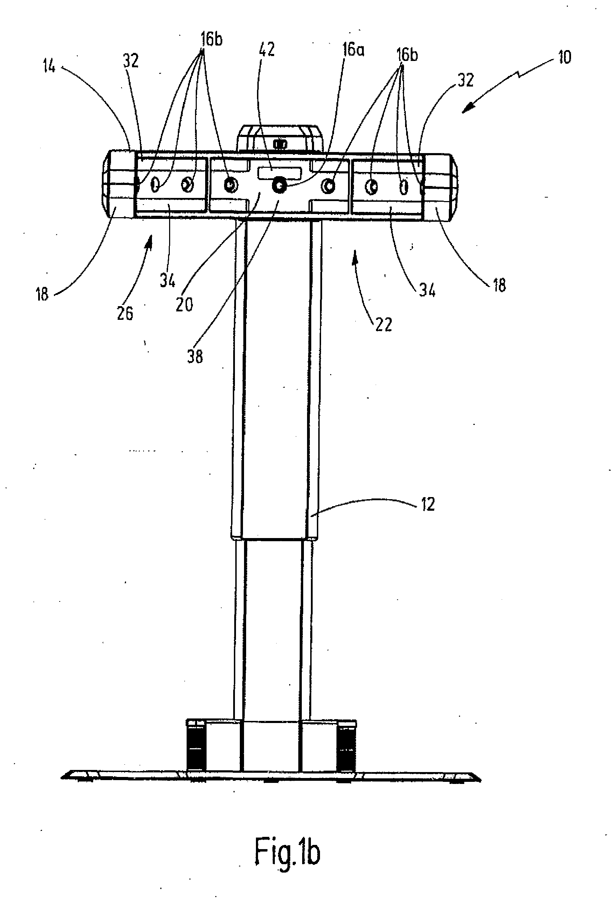

- Figur 1a, b: eine Vorrichtung zur Bestimmung von Zentrierparametern in perspektivischer Ansicht und in einer Ansicht von vorne;
- Figur 2: eine Veranschaulichung der Korrekturrechnung bei pupillenbasierter Bestimmung der Position des Hornhautscheitels und
- Figur 3a,b: eine Veranschaulichung einer nicht zur Erfindung gehörenden alternativen Korrekturrechnung bei reflexbasierter Bestimmung der Position des Hornhautscheitels.
Die in der Zeichnung dargestellte Vorrichtung 10 dient der Bestimmung von Zentrierparametern für die Brillenanpassung. Sie weist eine Säule 12 auf, die höhenverstellbar einen Kameraträger 14 trägt, welcher wiederum eine Anzahl Kameras 16a, 16b trägt. Der Kameraträger 14 ist in Draufsicht näherungsweise kreisförmig gebogen und erstreckt sich zwischen zwei freien Enden 18, welche im Abstand zueinander angeordnet sind. Nach vorne, also zur Säule 12 hin, und zu den Seiten umschließt eine Innenfläche 20 des Kameraträgers 14 einen Innenraum 22, in dem sich bei der Aufnahme von Bildern durch die Kameras 16a, 16b der Kopf eines Probanden befindet. Die Innenfläche 20 ist in einer Richtung, die zwischen den freien Enden 18 verläuft, konkav gebogen und weist beispielsweise die Form eines Abschnitts einer Zylindermantelfläche auf, wobei der Zylinder eine kreisrunde oder ovale Grundfläche haben kann. Um den Kameraträger 14 bezüglich des Kopfs des Probanden auf der richtigen Höhe positionieren zu können, ist in der Säule 12 eine nicht näher dargestellte Hubeinrichtung angeordnet, mit der der Kameraträger 14 motorisch angetrieben auf und ab bewegt werden kann.
Alle Kameras 16a, 16b sind in einer sich zwischen den freien Enden 18 erstreckenden Kameraanordnung 26 angeordnet. Im vorliegenden Ausführungsbeispiel ist die Kameraanordnung 26 als Kamerareihe 26 ausgebildet, deren Kameras 16a, 16b sich alle in derselben Höhe befinden, wobei ihre optischen Achsen auf den Innenraum 22 gerichtet sind. Im vorliegenden Ausführungsbeispiel umfasst die Kamerareihe 26 eine in der Mitte des Kameraträgers 14 angeordnete Frontkamera 16a, deren optische Achse frontal auf das Gesicht des Probanden gerichtet ist, sowie acht paarweise symmetrisch bezüglich einer durch die optische Achse der Frontkamera 16a verlaufenden senkrechten Symmetrieebene angeordnete Seitenkameras 16b von denen jeweils vier von links und von rechts auf das Gesicht des Probanden gerichtet sind. Die Kameras 16a, 16b sind zudem kalibriert, so dass sie gleichzeitig kalibrierte Bilder des Probanden aufnehmen können. Die Kalibrierung umfasst die extrinsischen Eigenschaften wie die relative Ausrichtung ihrer optischen Achsen sowie die relative Anordnung zueinander im Raum, sowie ihre intrinsischen Eigenschaften, also die Eigenschaften der Kameras selbst, die definieren, wie ein Punkt im Raum, der sich im internen Koordinatensystem der jeweiligen Kamera befindet, auf die Koordinaten der Pixel des aufgenommenen Bildes abgebildet wird.

Der Kameraträger 14 umschließt den Innenraum 22 nur nach vorne, zur Säule 12 hin, und zu den Seiten, also links und rechts des Kopfes des Probanden. Nach oben, unten sowie zu einer Rückseite 30 hin ist er offen, wobei die freien Enden 18 zueinander einen Abstand von mindestens 25cm aufweisen, so dass sich der Proband bequem von der Rückseite aus nähern kann. Im gezeigten Ausführungsbeispiel beträgt der Abstand 70 bis 80cm.

Zur Ausleuchtung des Innenraums 22 ist eine Beleuchtungseinrichtung mit einer oberhalb der Kamerareihe 26 verlaufenden oberen Lichtleiste 32 sowie einer unterhalb der Kamerareihe 26 verlaufenden unteren Lichtleiste 34 vorgesehen, welche jeweils eine Vielzahl von LEDs als Leuchtmittel aufweisen. Die obere Lichtleiste 32 und die untere Lichtleiste 34 erstrecken sich jeweils durchgehend oder mit Unterbrechungen über eine Länge, die mindestens so groß ist wie die Länge der in Umfangsrichtung zwischen den freien Enden 18 gemessenen Länge der Kamerareihe 26. Diese entspricht einem Umfangswinkel von mindestens 160 Grad. Nahe den freien Enden 18 sind die obere Lichtleiste 32 und die untere Lichtleiste 34 jeweils mittels einer vertikal verlaufenden weiteren Lichtleiste 36 miteinander verbunden. Die Kamerareihe 26 wird somit vollständig durch mindestens eine Reihe LEDs umrahmt. Die Vorrichtung 10 weist zudem eine in der Zeichnung nicht näher dargestellte Steuer- oder Regeleinrichtung auf, mit der die von den LEDs abgestrahlte Lichtintensität abhängig von der durch die Kameras 16a, 16b detektierten Lichtintensität gesteuert oder geregelt werden kann. Die LEDs der Lichtleisten 32, 34, 36 sind dabei zu Sektoren zusammengefasst, deren abgestrahlte Lichtintensitäten getrennt voneinander gesteuert bzw. geregelt werden können. Zudem ist vorgesehen, dass auch die von den einzelnen LEDs abgestrahlten Lichtintensitäten mittels der Steuer- oder Regeleinrichtung getrennt voneinander gesteuert oder geregelt werden können.

Um den Probanden richtig im Innenraum 22 positionieren zu können, sind die beiden der Frontkamera 16a nächstgelegenen Seitenkameras 16b dazu eingerichtet, den Abstand des Kopfs des Probanden von der Mitte 38 des Kameraträgers 14 zu messen. Mittels einer nicht näher dargestellten Anzeigeeinheit wird dem Probanden angezeigt, ob er richtig steht oder nicht. Die Anzeigeeinheit weist mehrere unterschiedlich eingefärbte Lichtquellen auf, die in einer Reihe angeordnet sind. Die mittlere Lichtquelle leuchtet grün, wenn der Proband richtig steht. Ausgehend von der mittleren Lichtquelle gibt es in jeder Richtung in dieser Reihenfolge eine gelbe, eine orangene und eine rote Lichtquelle, die entsprechend der Farbe anzeigt, wenn der Proband ein wenig, deutlich oder viel zu weit von der Mitte 38 des Kameraträgers 14 entfernt bzw. ein wenig, deutlich oder viel zu nah zur Mitte 38 steht. Um bei der Bestimmung der Zentrierparameter sicherzustellen, dass die Blickrichtung des Probanden ins Unendliche gerichtet ist, ist eine am Kameraträger 14 angeordnete Fixationseinrichtung 42 vorgesehen, die ein Fixationsmuster für den Probanden in Form eines Specklemusters erzeugt. Das Fixationsmuster ist etwas höher angeordnet als die Frontkamera 16a, so dass der Proband über diese hinwegblickt. Damit kann sein Gesicht im größtmöglichen Umfang aufgenommen werden.

Die Vorrichtung 10 eignet sich insbesondere auch zur Herstellung eines Avatars des Kopfs des Probanden, welcher zur Bestimmung der Zentrierparameter herangezogen werden kann. Zum diesem Zweck werden durch die Kameras 16a, 16b kalibrierte Bilder des Kopfs des Probanden ohne Brille bzw. Brillenfassung aufgenommen. Mittels eines geeigneten Prozesses zur geometrischen Positionsbestimmung wie beispielsweise Triangulation wird ein Tiefenprofil des Kopfes erstellt, das diesen näherungsweise sehr gut abbildet. Der Kopf wird durch eine Vielzahl von Punkten abgebildet, die mittels eines Netzmusters miteinander verbunden werden können oder aber als Punktwolke gespeichert werden können. Bei der anschließenden Bestimmung der Zentrierparameter kann der so ermittelte Avatar herangezogen werden, um Zentrierparameter zu bestimmen, die aufgrund der geometrischen Eigenschaften der Brille bzw. Brillenfassung, die der Proband trägt, nicht oder nur näherungsweise bestimmt werden können. Beispielsweise kann ein breiter Fassungsbügel das Auge in einer Seitenaufnahme soweit verdecken, dass der Hornhautscheitelabstand nicht oder nur sehr ungenau bestimmt werden kann. Zudem können gefärbte oder stark spiegelnde Gläser die Augen nicht oder nur sehr schlecht erkennen lassen. Um dem zu begegnen, wird auf die von den Kameras 16a, 16b aufgenommenen Bilder des die Brille oder Brillenfassung tragenden Probanden das Tiefenprofil des Avatars projiziert und die Zentrierparameter, die aufgrund der durch die Brille bzw. Brillenfassung eingeschränkten Sicht nur ungenügend bestimmt werden können, werden mittels der Bilddaten des Avatars bestimmt. Dabei kann eine Anpassung des Avatars auf die Bilder des die Brille bzw. Brillenfassung tragenden Probanden zur Minimierung von Abweichungen erfolgen.

Die oben beschriebene Vorrichtung 10 kann wie folgt für eine pupillenbasierte Detektion eines Hornhautscheitels ebenso wie für eine reflexbasierte Detektion eines Hornhautscheitels bei beiden Augen des Probanden eingesetzt werden.

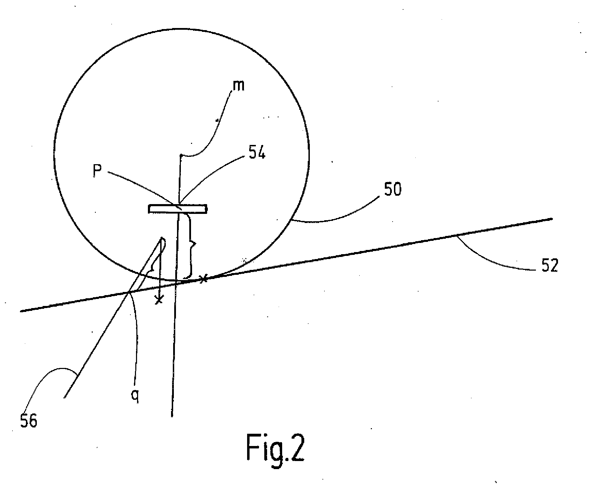

Bei der erfindungsgemäßen pupillenbasierten Methode gemäß Figur 2 wird zunächst die Position des Hornhautscheitels im Raum in erster Näherung als Schnittpunkt q eines die Hornhaut 50 tangierenden ersten Sichtstrahls 52 einer der ein Seitenbild des Probanden aufnehmenden Seitenkameras 16b mit einem auf die Pupille 54 gerichteten zweiten Sichtstrahl 56 der ein Frontbild des Probanden aufnehmenden Frontkamera 16a bestimmt. Mittels einer Korrekturrechnung wird eine korrigierte Position des Hornhautscheitels im Raum mittels der Gleichung a = q + µ * v + µ * w berechnet. Dabei ist µ ein Erfahrungswert oder ein tatsächlicher Wert für den Abstand zwischen dem Pupillenzentrum und dem Hornhautscheitel, der regelmäßig Werte zwischen 2,5mm und 4mm annimmt. v ist ein Einheitsvektor der Raumrichtung vom Pupillenzentrum p zur Frontkamera 16a, deren Koordinaten mit der Variable c1 angegeben werden, und errechnet sich als v = (p-c1) / | p - c1 |. w ist ein Einheitsvektor der durch das Zentrum m der Hornhautkugel verlaufenden Blickrichtung, die auf das Fixationsmuster der Fixationseinrichtung 42 am Raumpunkt t gerichtet ist, und errechnet sich zu w = (t - m) / | t - m |. Alle Werte a, q, p, c1, t und m sind dreidimensionale Vektoren.

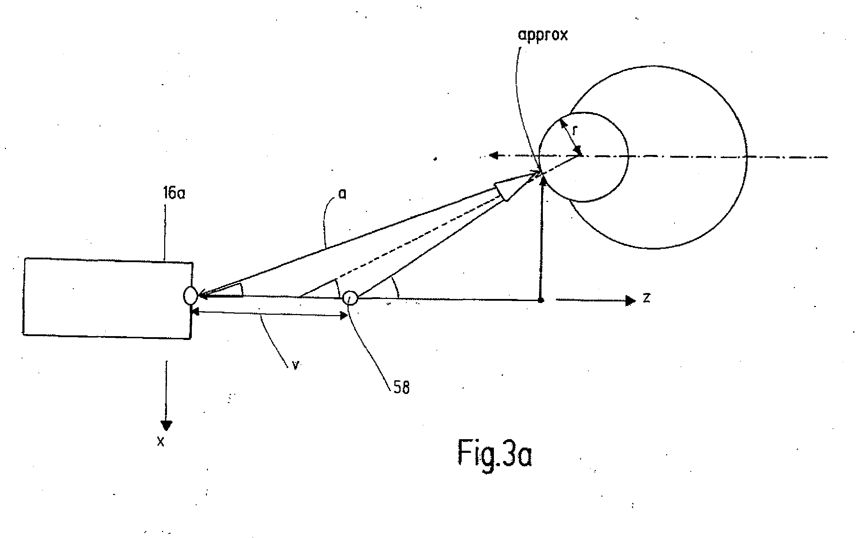

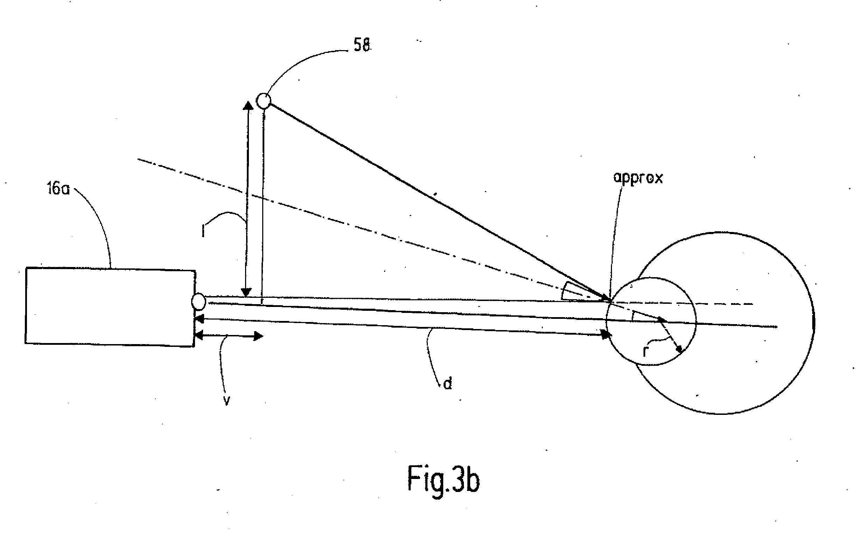

Bei der nicht zur Erfindung gehörenden alternativen reflexbasierten Bestimmung der Position des Hornhautscheitels gemäß Figur 3a, 3b sind zwei Korrekturrechnungen vorzunehmen, wobei die erste Korrekturrechnung (Figur 3a) eine Korrektur in x-Richtung, die zweite Korrektur (Figur 3b) eine Korrektur in y-Richtung betrifft. Diese Raumrichtungen werden durch ein inneres Koordinatensystem der Frontkamera 16a festgelegt, das seinen Nullpunkt im optischen Zentrum der Frontkamera 16a hat. Die z-Richtung wird dabei durch die Blickrichtung der Frontkamera 16a festgelegt, die x-Richtung ist eine Richtung, die horizontal sowie orthogonal zur z-Richtung verläuft und in deren Richtung betrachtet nach rechts weist, und die y-Richtung verläuft orthogonal zur x-Richtung und zur z-Richtung und weist im Raum nach oben. Bei der reflexbasierten Messung wird mittels einer Lichtquelle, im vorliegenden Fall einer LED 58 ein Lichtblitz ausgesandt, dessen Reflexion auf der Hornhaut von der Frontkamera 16a und mindestens einer der Seitenkameras 16b detektiert wird und die erste Näherung für die Position des Hornhautscheitels im Raum bildet. Der Reflexpunkt wird in Figur 3a, 3b mit "approx" bezeichnet. In x-Richtung wird eine Korrektur durch Addition von Δx =+/ -r * sin (½ * (arccos z/a + arctan x/(z-v))) zur x-Koordinate des Reflexpunkts approx vorgenommen, wobei das Pluszeichen bei der Anwendung auf das linke Auge, das Minuszeichen bei der Anwendung auf das rechte Auge (vgl. Figur 3a) zu verwenden ist. r ist dabei ein Erfahrungswert oder ein tatsächlicher Wert für den Hornhautradius, der typischerweise ca. 8mm beträgt. a ist der Abstand des optischen Zentrums der Frontkamera 16a zum Reflexpunkt approx und v ist der Abstand der LED 58 zum optischen Zentrum der Frontkamera 16a in z-Richtung. x und z wiederum sind die Koordinaten in x- und z-Richtung.
In y-Richtung wird ausgehend vom Reflexpunkt approx eine Korrektur mittels Addition von Δy =+/ -r * sin ½ * (arctan l/(d-v)) vorgenommen. r ist wiederum der Erfahrungswert oder ein tatsächlicher Wert für den Hornhautradius, d ist der Abstand des optischen Zentrums der Frontkamera 16a zum Reflexpunkt approx in z-Richtung, v ist der Abstand der LED 58 zum optischen Zentrum der Frontkamera 16a in z-Richtung und l ist der Abstand der LED 58 vom optischen Zentrum der Frontkamera 16a in y-Richtung. Das Pluszeichen kommt zur Anwendung, wenn die LED 58 unter der Frontkamera 16a angeordnet ist, also ihre y-Koordinate kleiner ist als die y-Koordinate der Frontkamera 16a bzw. ihres optischen Zentrums. Ist die LED über der Frontkamera 16a angeordnet, so kommt das Minuszeichen zum Einsatz.

Bei den beschriebenen Verfahren kann die Pupille bzw. der Reflexpunkt approx beispielsweise mittels Merkmalsextraktion, mittels Merkmalsmatching und/oder mittels maschinellem Lernen durch Vergleich mit einer Vielzahl vorbekannter Daten detektiert werden. Diesem Detektionsschritt kann ein Schritt vorangehen, bei dem ein Gesichtsdetektor erkennt, welche Bildpunkte zum Gesicht des Probanden bzw. zu seiner Augenpartie gehören, so dass nach der Pupille bzw. dem Reflexpunkt approx bereits eingegrenzt gesucht werden kann.

## Patentansprüche

1. Computerimplementiertes Verfahren zur Detektion eines Hornhautscheitels, wobei ein Frontbild eines Kopfes und ein Seitenbild des Kopfes bereitgestellt werden, die zueinander kalibriert sind, wobei aus dem Frontbild und dem Seitenbild mittels geometrischer Positionsbestimmung die Position des Hornhautscheitels im Raum bestimmt wird, wobei die Position des Hornhautscheitels einer Korrekturrechnung unterzogen wird und wobei die Position des Hornhautscheitels im Raum in einer ersten Näherung als Schnittpunkt q eines die Hornhaut (50) tangierenden ersten Sichtstrahls (52) einer das Seitenbild aufnehmenden Seitenkamera (16b) mit einem auf die Pupille (54) gerichteten zweiten Sichtstrahl (56) einer das Frontbild aufnehmenden Frontkamera (16a) bestimmt wird, **dadurch gekennzeichnet, dass** mittels der Korrekturrechnung die Position des Hornhautscheitels gemäß a = q + µ * v + µ * w berechnet wird, wobei a der Positionsvektor des Hornhautscheitels im Raum nach Durchführung der Korrekturrechnung, q die Position des Hornhautscheitels in erster Näherung, µ ein Erfahrungswert oder ein tatsächlicher Wert für den Abstand zwischen einem Pupillenzentrum und dem Hornhautscheitel, v ein Einheitsvektor der Raumrichtung vom Pupillenzentrum zur Frontkamera und w ein Einheitsvektor der durch das Zentrum einer Hornhautkugel verlaufenden Blickrichtung ist.

2. Computerimplementiertes Verfahren nach Anspruch 1, **dadurch gekennzeichnet, dass** bei der geometrischen Positionsbestimmung ein Triangulationsverfahren verwendet wird.

3. Computerimplementiertes Verfahren nach einem der vorangehenden Ansprüche, **dadurch gekennzeichnet, dass** die Pupille bzw. der Reflexpunkt mittels Merkmalsextraktion und/oder Merkmalsmatchings und/oder mittels Maschinellen Lernens durch Vergleich mit einer Vielzahl vorbekannter Daten detektiert wird.

4. Computerimplementiertes Verfahren nach einem der vorangehenden Ansprüche, **dadurch gekennzeichnet, dass** aus dem Frontbild ein auf den Hornhautscheitel gerichteter Sichtstrahl der Frontkamera (16a) berechnet und in das Seitenbild projiziert wird und dass der Hornhautscheitel auf dem ins Seitenbild projizierten Sichtstrahl detektiert wird.

5. Computerimplementiertes Verfahren nach einem der vorangehenden Ansprüche, **dadurch gekennzeichnet, dass** mindestens ein gleichzeitig mit dem ersten und zweiten Bild seitlich bezüglich des Kopfes aufgenommenes, kalibriertes drittes Bild bereitgestellt wird.

6. Verfahren zur Bestimmung eines Hornhautscheitelabstands eines eine Brillenfassung oder eine Brille tragenden Probanden, **dadurch gekennzeichnet, dass** die Position des Hornhautscheitels im Raum mittels eines Verfahrens nach einem der vorangehenden Ansprüche bestimmt wird und dass die Position des Hornhautscheitels im Raum für die Bestimmung des Hornhautscheitelabstands herangezogen wird.

7. Computerprogramm mit Programmcode zur Durchführung aller Verfahrensschritte nach einem der Ansprüche 1 bis 5, wenn das Computerprogramm in einem Computer geladen und in einem Computer ausgeführt wird.

8. Verwendung einer Vorrichtung (10) zur Durchführung eines computerimplementierten Verfahrens nach einem der Ansprüche 1 bis 5, wobei die Vorrichtung (10) einen Kameraträger (14) aufweist, welcher einen nach oben, nach unten und zu einer Rückseite (30) hin offenen Innenraum (22) teilweise umschließt und mindestens drei Kameras (16a, 16b) trägt, die zwischen zwei freien Enden (18) des Kameraträgers (14) angeordnet und auf den Innenraum (22) gerichtet sind, wobei der Kameraträger (14) zur Beleuchtung des Innenraums (22) eine Beleuchtungseinrichtung (32, 34, 36) aufweist.

9. Vorrichtung zur Detektion eines Hornhautscheitels mit einer Frontkamera (16a) zur Aufnahme eines Frontbilds eines Probanden, mit einer Seitenkamera (16b) zur Aufnahme eines Seitenbilds des Probanden, wobei die Frontkamera und die Seitenkamera zueinander kalibriert sind, so dass das Frontbild und das Seitenbild zueinander kalibriert sind, und mit einem Computer, welcher einen Prozessor aufweist, der eingerichtet ist, aus dem Frontbild und dem Seitenbild mittels geometrischer Positionsbestimmung die Position eines Hornhautscheitels im Raum zu bestimmen und die Position des Hornhautscheitels im Raum einer Korrekturrechnung zu unterziehen, wobei die Position des Hornhautscheitels im Raum in einer ersten Näherung als Schnittpunkt q eines die Hornhaut (50) tangierenden ersten Sichtstrahls (52) einer das Seitenbild aufnehmenden Seitenkamera (16b) mit einem auf die Pupille (54) gerichteten zweiten Sichtstrahl (56) einer das Frontbild aufnehmenden Frontkamera (16a) bestimmt wird, **dadurch gekennzeichnet, dass** mittels der Korrekturrechnung die Position des Hornhautscheitels gemäß a = q + µ * v + µ * w berechnet wird, wobei a der Positionsvektor des Hornhautscheitels im Raum nach Durchführung der Korrekturrechnung, q die Position des Hornhautscheitels in erster Näherung, µ ein Erfahrungswert oder ein tatsächlicher Wert für den Abstand zwischen einem Pupillenzentrum und dem Hornhautscheitel, v ein Einheitsvektor der Raumrichtung vom Pupillenzentrum zur Frontkamera und w ein Einheitsvektor der durch das Zentrum einer Hornhautkugel verlaufenden Blickrichtung ist.

## Claims

1. Computer-implemented method for detecting a corneal vertex, wherein a frontal image of a head and a lateral image of the head are provided, said images being calibrated to one another, wherein the position of the corneal vertex in space is determined from the frontal image and the lateral image by means of a geometric position determination, wherein the position of the corneal vertex is subjected to a correction calculation and wherein the position of the corneal vertex in space is determined to a first approximation as the point of intersection q of a first view ray (52), tangential to the cornea (50), from a lateral camera (16b) recording the lateral image with a second view ray (56), directed onto the pupil (54), from a frontal camera (16a) recording the frontal image, **characterized in that** the position of the corneal vertex is calculated according to a = q + µ * v + µ * w by means of the correction calculation, where a denotes the position vector of the corneal vertex in space after carrying out the correction calculation, q denotes the position of the corneal vertex to a first approximation, µ denotes an empirical value or an actual value for the distance between a pupil centre and the corneal vertex, v denotes a unit vector in the spatial direction from the pupil centre to the frontal camera and w denotes a unit vector in the viewing direction extending through the centre of a corneal sphere.

2. Computer-implemented method according to Claim 1, **characterized in that** a triangulation method is used in the geometric position determination.

3. Computer-implemented method according to either of the preceding claims, **characterized in that** the pupil or the reflection point is detected by means of feature extraction and/or feature matching and/or by means of machine learning by comparison with a multiplicity of data known in advance.

4. Computer-implemented method according to any one of the preceding claims, **characterized in that** a view ray of the frontal camera (16a) directed to the corneal vertex is calculated from the frontal image and said view ray is projected into the lateral image, and **in that** the corneal vertex is detected on the view ray that is projected into the lateral image.

5. Computer-implemented method according to any one of the preceding claims, **characterized in that** provision is made of at least one calibrated third image that is recorded from a lateral position in respect of the head at the same time as the first and second image.

6. Method for determining a vertex distance of a spectacle frame or a spectacles-wearing subject, **characterized in that** the position of the corneal vertex in space is determined by means of a method according to any one of the preceding claims and **in that** the position of the corneal vertex in space is used for determining the vertex distance.

7. Computer program with program code for carrying out all of the method steps according to any one of Claims 1 to 5, when the computer program is loaded onto a computer and/or executed on a computer.

8. Use of an apparatus (10) for carrying out a computer-implemented method according to any one of Claims 1 to 5, wherein the apparatus (10) has a camera carrier (14), which partly encloses an interior (22) that is open to the top, to the bottom and to a rear side (30) and which carries at least three cameras (16a, 16b) which are arranged between two free ends (18) of the camera carrier (14) and directed onto the interior (22), wherein the camera carrier (14) has an illumination device (32, 34, 36) for illuminating the interior (22).

9. Apparatus for detecting a corneal vertex, having a frontal camera (16a) for recording a frontal image of a subject, having a lateral camera (16b) for recording a lateral image of the subject, wherein the frontal camera and the lateral camera are calibrated to one another such that the frontal image and the lateral image are calibrated to one another, and having a computer, which has a processor configured to determine the position of a corneal vertex in space from the frontal image and the lateral image by means of a geometric position determination and to subject the position of the corneal vertex in space to a correction calculation, wherein the position of the corneal vertex in space is determined to a first approximation as the point of intersection q of a first view ray (52), tangential to the cornea (50), from a lateral camera (16b) recording the lateral image with a second view ray (56), directed onto the pupil (54), from a frontal camera (16a) recording the frontal image, **characterized in that** the position of the corneal vertex is calculated according to a = q + µ * v + µ * w by means of the correction calculation, where a denotes the position vector of the corneal vertex in space after carrying out the correction calculation, q denotes the position of the corneal vertex to a first approximation, µ denotes an empirical value or an actual value for the distance between a pupil centre and the corneal vertex, v denotes a unit vector in the spatial direction from the pupil centre to the frontal camera and w denotes a unit vector in the viewing direction extending through the centre of a corneal sphere.

## Revendications

1. Procédé mis en oeuvre par ordinateur pour détecter un sommet de la cornée, dans lequel une image frontale d'une tête et une image latérale de la tête, qui sont calibrées l'une par rapport à l'autre, sont fournies, dans lequel la position du sommet de la cornée dans l'espace est déterminée à partir de l'image frontale et de l'image latérale au moyen d'une détermination géométrique de la position, dans lequel la position du sommet de la cornée est soumise à un calcul de correction et dans lequel la position du sommet de la cornée dans l'espace est déterminée en première approximation comme étant le point d'intersection q d'un premier faisceau visuel (52) tangent à la cornée (50) d'une caméra latérale (16b) qui acquiert l'image latérale avec un second faisceau visuel (56) dirigé vers la pupille (54) d'une caméra frontale (16a) qui acquiert l'image frontale, **caractérisé en ce que** la position du sommet de la cornée est calculée au moyen du calcul de correction suivant : a = q + µ * v + µ * w, où a est le vecteur de position du sommet de la cornée dans l'espace après que le calcul de correction a été effectué, q est la position du sommet de la cornée en première approximation, µ est une valeur empirique ou une valeur réelle de la distance entre le centre de la pupille et le sommet de la cornée, v est un vecteur unitaire de la direction spatiale du centre de la pupille à la caméra avant et w est un vecteur unitaire de la direction du regard passant par le centre d'une sphère cornéenne.

2. Procédé mis en oeuvre par ordinateur selon la revendication 1, **caractérisé en ce qu'**un procédé de triangulation est utilisé pour la détermination géométrique de la position.

3. Procédé mis en oeuvre par ordinateur selon l'une des revendications précédentes, **caractérisé en ce que** la pupille ou le point de réflexion est détecté par extraction de caractéristiques et/ou reconnaissance de caractéristiques et/ou par apprentissage machine par comparaison avec une pluralité de données précédemment connues.

4. Procédé mis en oeuvre par ordinateur selon l'une des revendications précédentes, **caractérisé en ce qu'**un faisceau visuel de la caméra frontale (16a) dirigé vers le sommet de la cornée est calculé à partir de l'image frontale et projeté dans l'image latérale et **en ce que** le sommet de la cornée est détecté sur le faisceau visuel projeté dans l'image latérale.

5. Procédé mis en oeuvre par ordinateur selon l'une des revendications précédentes, **caractérisé en ce qu'**il est prévu au moins une troisième image calibrée qui est prise simultanément avec la première et la deuxième images latéralement par rapport à la tête.

6. Procédé pour déterminer la distance du sommet de la cornée d'une personne de test portant une monture de lunettes, **caractérisé en ce que** la position du sommet de la cornée dans l'espace est déterminée au moyen d'un procédé selon l'une des revendications précédentes et **en ce que** la position du sommet de la cornée dans l'espace est utilisée pour déterminer la distance du sommet de la cornée.

7. Programme d'ordinateur comportant un code de programme destiné à mettre en oeuvre toutes les étapes selon l'une des revendications 1 à 5 lorsque le programme d'ordinateur est chargé dans un ordinateur et exécuté sur un ordinateur.

8. Utilisation d'un dispositif (10) destiné à réaliser un procédé mis en oeuvre par ordinateur selon l'une des revendications 1 à 5, dans lequel le dispositif (10) comprend un support de caméra (14) entourant partiellement un espace intérieur (22) ouvert vers le haut, vers le bas et vers une face arrière (30) et portant au moins trois caméras (16a, 16b) disposées entre deux extrémités libres (18) du support de caméra (14) et dirigées vers l'espace intérieur (22), dans lequel le support de caméra (14) comprend des moyens d'éclairement (32, 34, 36) pour éclairer l'espace intérieur (22).

9. Dispositif de détection d'un sommet de la cornée comportant une caméra avant (16a) pour l'acquisition d'une image frontale d'un sujet, une caméra latérale (16b) l'acquisition la prise d'une image latérale du sujet, dans lequel la caméra avant et la caméra latérale sont calibrées l'une par rapport à l'autre de manière à ce que l'image avant et l'image latérale soient calibrées l'une par rapport à l'autre, et comportant un ordinateur qui comporte un processeur conçu pour déterminer la position d'un sommet de la cornée dans l'espace à partir de l'image frontale et de l'image latérale au moyen d'une détermination géométrique de la position d'un sommet de la cornée dans l'espace et pour soumettre la position du sommet de la cornée dans l'espace à un calcul de correction, dans lequel la position du sommet de la cornée dans l'espace est déterminée en première approximation comme étant le point d'intersection q d'un premier faisceau visuel (52) tangent à la cornée (50) d'une caméra latérale (16b) qui acquiert l'image latérale avec un deuxième faisceau visuel (56) dirigé vers la pupille (54) d'une caméra avant (16a) qui acquiert l'image frontale,
**caractérisé en ce que** la position du sommet de la cornée est calculée au moyen du calcul de correction suivant : a = q + µ * v + µ * w, où a est le vecteur de position du sommet de la cornée dans l'espace après que le calcul de correction a été effectué, q est la position du sommet de la cornée en première approximation, µ est une valeur empirique ou une valeur réelle de la distance entre le centre de la pupille et le sommet de la cornée,
v est un vecteur unitaire de la direction spatiale du centre de la pupille à la caméra avant et w est un vecteur unitaire de la direction du regard qui passe par le centre d'une sphère cornéenne.
